# EUROPÄISCHE PATENTSCHRIFT

(11) **EP 0 815 320 B1**
(45) Veröffentlichungstag und Bekanntmachung des Hinweises auf die Patenterteilung: **02.06.1999**
(21) Anmeldenummer: 96905746.2
(22) Anmeldetag: 19.03.1996
(51) Int. Cl.: D21G 9/00, G01N 33/34

(54) **VERFAHREN UND VORRICHTUNG ZUR PROZESSFÜHRUNG EINER PAPIERMASCHINE**
METHOD AND DEVICE FOR PROCESS MANAGEMENT IN PAPER AND CARDBOARD MANUFACTURE
PROCEDE ET DISPOSITIF DE REGULATION DU PROCESSUS D'EXPLOITATION D'UNE MACHINE A PAPIER

(30) Priorität: 23.03.1995 DE 19510009
(43) Veröffentlichungstag der Anmeldung: 07.01.1998
(73) Patentinhaber: SIEMENS AKTIENGESELLSCHAFT, 80333 München (DE)
(72) Erfinder: FURUMOTO, Herbert, D-91052 Erlangen (DE); GERDEMANN, Ulrich, D-91094 Langensendelbach (DE); ZEINER, Gerhard, D-70563 Stuttgart (DE)
(86) Internationale Anmeldenummer: DE9600476
(87) Internationale Veröffentlichungsnummer: WO9629468

(56) Entgegenhaltungen:
- EP-A- 0 137 696
- WO-A-95/08019
- US-A- 4 098 641
- US-A- 5 220 172
- US-A- 5 282 131

## Beschreibung

Die Erfindung bezieht sich auf ein Verfahren und eine Vorrichtung zur Prozeßführung einer Papiermaschine für die Papier- und/oder Kartonherstellung unter Verwendung wenigstens einer Meßeinrichtung zur Erfassung physikalischer Kennwerte und wenigstens einer Regel- oder Steuereinrichtung für die bei der Papiermaschine eingesetzten Betriebsmittel.

Mit der älteren, nicht vorveröffentlichten internationalen Patentanmeldung WO 95/08019 A1 wird eine Vorrichtung zum Betrieb einer Anlage speziell zur Erzeugung von deinktem Zellstoff vorgeschlagen, welche zumindest eine Altpapieraufbereitung enthält, der eine Papiermaschine oder mindestens eine Entwässerungsmaschine nachgeschaltet ist. Dabei werden bereits Meßeinrichtungen zur Erfassung spektraler und/ oder physikalischer Kennwerte der Altpapiersuspension verwendet. Weiterhin sind dort Regel- oder Steuereinrichtungen für die Betriebsmittel der Altpapieraufbereitung verwendet und ist mindestens ein, in Form eines oder mehrerer paralleler neuronaler Netze ausgeführter Zustandsanalysator für die Altpapiersuspension vorhanden, welcher mittels der Kennwerte der Meßeinrichtung Steuergrößen zur Prozeßführung an den Regel- oder Steuereinrichtungen der Betriebsmittel für die Altpapieraufbereitung liefert.

Bei der vorbeschriebenen Vorrichtung zur Herstellung von deinktem Zellstoff unter Verwendung eines möglichst großen Anteiles von Altpapier besteht das Problem insbesondere darin, daß die Qualität von in die Anlage eingebrachten Altpapieren stark schwankt. Beispielsweise können von der jeweiligen Mischung des Altpapieres stark veränderliche Anteile z.B. an bunten illustrierten Papieren, grauen Zeitungspapieren, weißen Papieren, verschmutzten Papieren, Altbüchern z.B. mit Kleberücken, wie z.B. Telefonbücher, Kartonagen, Verpackungen, beschichteten Papieren sowie Verschmutzungen aller Art vorhanden sein. Die in der älteren Patentanmeldung vorbeschriebene Vorrichtung löst diese Probleme in befriedigender Weise für die Altpapieraufbereitung.

Aus der EP-A-0 137 696 ist ein Verfahren und eine zugehörige Vorrichtung zur Erfassung des Wassergehaltes einer Papierbahn während der Herstellung bekannt, bei dem über eine optische Infrarot-Messung ausgenutzt wird, daß das Wasser eine Absorptionsbande bei 1,94 µm hat. Dazu wird ein Meßkanal und ein Referenzkanal mit einer anderen Wellenlänge als die Wasserabsorptionsbande verwendet. Daneben ist aus der US-A-5 282 131 ein System zur Regelung einer Zellstoffwaschanlage bekannt, bei der ein neuronales Netzwerk zur Verifizierung von vorhersagbaren Prozeßparametern eingesetzt wird. Bei beiden Druckschriften werden also jeweils nur Teilaspekte bei der Papierherstellung angesprochen.

Aufgabe vorliegender Erfindung ist es demgegenüber, das der vorbeschriebenen Vorrichtung zugrundeliegende Meßprinzip direkt bei einer Papiermaschine einzusetzen.

Die Aufgabe ist erfindungsgemäß dadurch gelöst, daß mit der Meßeinrichtung spektrale Kennwerte bei unterschiedlichen Wellenlängen an den Betriebsstoffen der Papiermaschine erfaßt werden, wobei die Betriebsstoffe der Ausgangsstoff unmittelbar vor dem Stoffauflauf an der Papiermaschine und/oder die Zwischen- bzw. Endprodukte sind, und daß die Signale der Meßeinrichtung für die spektralen Kennwerte von wenigstens einem neuronalen Netz bewertet und daraus Aussagen über die Produktqualität abgeleitet werden, woraus Signalgrößen einerseits zur Rückwärtsregelung bei der sogenannten Stoffaufbereitung vor der verwendeten Papiermaschine und andererseits zur Vorwärtsregelung der Papiermaschine selbst abgeleitet werden. Die Produktqualität ist dabei insbesondere die Qualitätsparameter des Papiers bzw. des Kartons.

Bei der Rückwärtsregelung werden die Parameter der Stoffaufbereitung derart eingestellt, um die für eine bestimmte Papier- oder Kartonqualität erforderliche Stoffqualität zu produzieren. Bei der Vorwärtsregelung werden dagegen die Betriebsmittel der Papiermaschine derart gesteuert, daß bei einer gegebenen Stoffqualität eine bestimmte Papier- oder Kartonqualität erreicht wird.

Mit der Erfindung ergibt sich erstmalig die Möglichkeit einer Online-Messung mit dem Spektrometer bei einer Papiermaschine. Durch geeignete Auswertung mit Bestimmung der Qualitätsmaßzahlen des Papiers oder auch des Kartons können somit auch die qualitätsbeeinflussenden Parameter bei der Stoffaufbereitung für die Papiermraschine beeinflußt werden. Die bei der bisher üblichen Labormessung entstehenden Verzögerungszeiten entfallen somit.

Bei der zugehörigen Anordnung werden Spektroskope bzw. Spektrometer insbesondere zur Aufnahme von spektralen Verteilungen oder Gesamtspektren verwendet, wobei im Rahmen der Erfindung die neuronalen Netze speziell zur Auswertung der spektralen Kennwerte eingesetzt werden. Als Eingangsgrößen für die neuronalen Netze wird vorzugsweise entweder die diffuse Rückstrahlintensität oder aber die diffuse Durchstrahlintensität ausgewählter Spektralbereiche benutzt. Vorteilhafterweise lassen sich weitere Parameter der Stoffsuspension oder des Papiers oder Kartons, beispielsweise Konsistenz, Feuchte, Flächengewicht und ähnliches als Eingangsgrößen für die neuronalen Netze verwenden. Ausgangsgrößen der neuronalen Netze sind mechanische Qualitätsparameter des produzierten Papiers oder Kartons, wie insbesondere der sogenannte CMT-Wert, die Reißlänge, der Berstdruck und andere für die Praxistauglichkeit des Papiers bedeutsame Faktoren. Die neuronalen Netze können mit im Labor offline gemessenen Qualitätsparametern trainiert werden.

Weitere Einzelheiten und Vorteile der Erfindung ergeben sich aus der nachfolgenden Figurenbeschreibung von Ausführungsbeispielen anhand der Zeichnung in Verbindung mit den Patentansprüchen. Es zeigen in schematischer Darstellung
- FIG 1: ein Anwendungsbeispiel mit einer Papiermaschine,
- FIG 2 und 3: jeweils zwei neuronale Teilnetze zur Verwendung bei FIG 1 und
- FIG 4 bis 7: Alternativen für den Einsatz von Spektrometern.

In den Figuren haben gleiche bzw. gleichwirkende Teile sich entsprechende Bezugszeichen. Die Figuren werden teilweise gemeinam beschrieben.

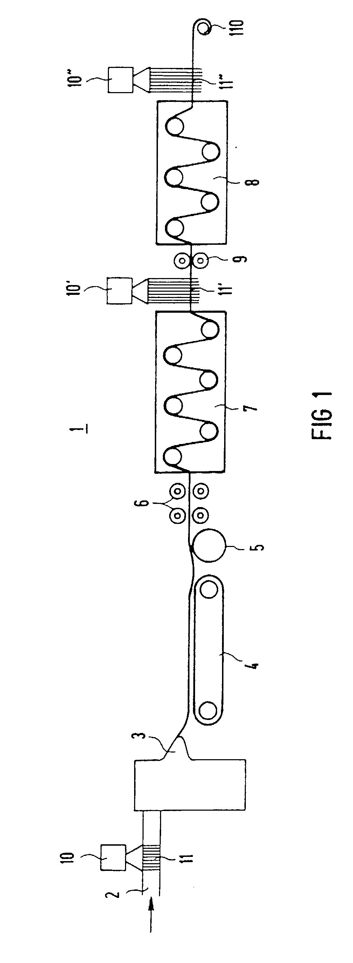

In FIG 1 ist eine Anlage zur Papiererzeugung mit 1 bezeichnet, die im wesentlichen aus dem sogenannten Stoffeinlauf 2, der eigentlichen Papiermaschine mit Stoffauflauf 3, einem nachfolgenden Siebförderband 4 zum Transport der feuchten Papierbahnen, weiteren Rollenbahnen 5 bis 8 zum Trocknen und Aufwickeln einer Papierbahn 110 sowie aus einer Leimpresse 9 besteht. Insbesondere die Leimpresse 9 kann zwischen Trocknungseinrichtungen 7 und 8 angeordnet sein. Derartige Anlagen zur Papiererzeugung sind in vielfältiger technischer Ausgestaltung bekannt und in der Praxis im Einsatz.

In der Anlage gemäß FIG 1 ist ein erstes Spektrometer 10 im Bereich des Stoffeinlaufes 1 vorhanden, dessen Meßfläche 11 auf die Stoffsuspension als Ausgangsstoff für die Papierherstellung gerichtet ist. Ein zweites Spektrometer 10' ist vor der Leimpresse 9 mit seiner Meßfläche 11' direkt auf die Papierbahn 110 gerichtet. Weiterhin ist ein drittes Spektrometer 10" mit seiner Meßfläche 11" nach der Leimpresse 9 auf das beleimte Papier vor der sich anschließenden, nicht im einzelnen dargestellten Aufwickelvorrichtung angeordnet.

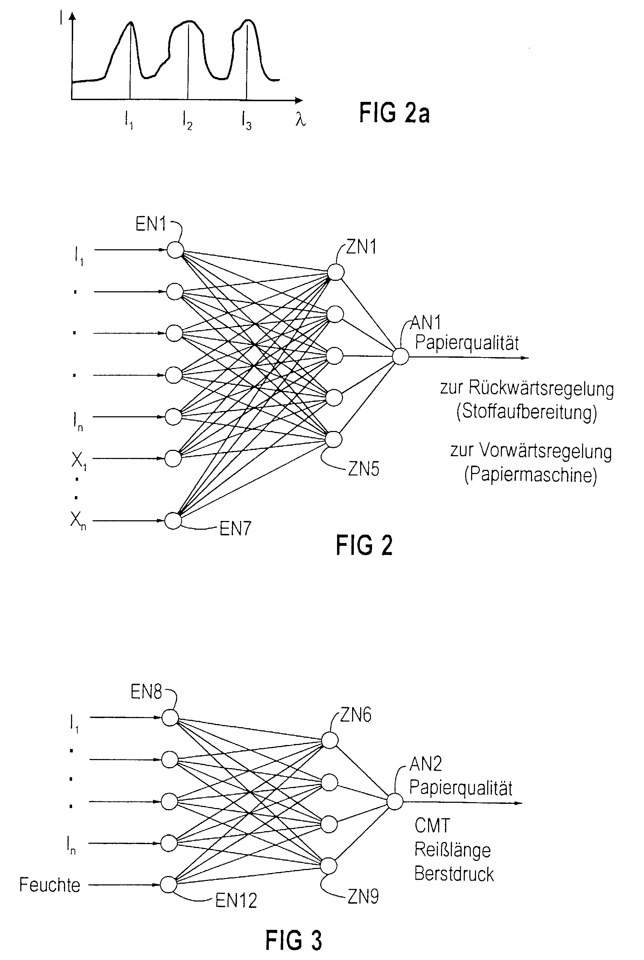

In FIG 2 ist ein dreischichtiges neuronales Netz mit 20 bezeichnet, das beispielhaft aus Eingangsneuronen EN1 bis EN7 sowie weiteren Neuronen ZN1 bis ZN5 und einem zugehörigen Ausgangsneuron AN1 besteht. Mit dem neuronalen Netz 20 wird das Spektrum des ersten Spektrometers 10 ausgewertet, wobei als Eingänge für das neuronale Netz 20 die Rückstrahlintensitäten ***I***_{***1***} bis ***I***_{***n***} von bevorzugten Wellenlängen **λ**_{**i**} des schematischen Diagramms in FIG 2a verwendet werden. Aus den Intensitäten ***I***_{**i**} mit i = 1,...,n lassen sich Aussagen über die Qualität des herzustellenden Papieres gewinnen. Diese können einerseits die Rückwärtsregelung bei der Stoffaufbereitung und andererseits zur Vorwärtsregelung bei der Papiermaschine 1 selbst verwendet werden.

In FIG 3 ist ein anderes dreischichtiges neuronales Netz 30 dargestellt, das Eingangsneuronen EN8 bis EN12 sowie weitere Neuronen ZN6 bis ZN9 und ein Ausgangsneuron AN2 hat. Mit diesem zweiten neuronalen Netz werden die Spektrometer 10' bzw. 10'' in entsprechender Weise wie bei FIG 2 ausgewertet Es empfiehlt sich als weitere Eingangsgröße die Feuchte der Papierbahn 110 als Meßgröße zu verwenden. Damit lassen sich Aussagen über die Produktqualität des fertigen Papiers oder des Kartons gewinnen. Weiterhin können mechanische Parameter wie der sogenannte CMT-Faktor, die Reißlänge und der Berstdruck erhalten werden.

Es ist auch möglich für die beiden weiteren Spektrometer 10' und 10'' je ein eigenes neuronales Teilnetz vorzusehen. Die neuronalen Netze gemäß den Figuren 2 und 3 können auch zusammengefaßt werden, wobei durch deren Verknüpfung die Sicherheit für die Ableitung der Meßgrößen verbessert wird.

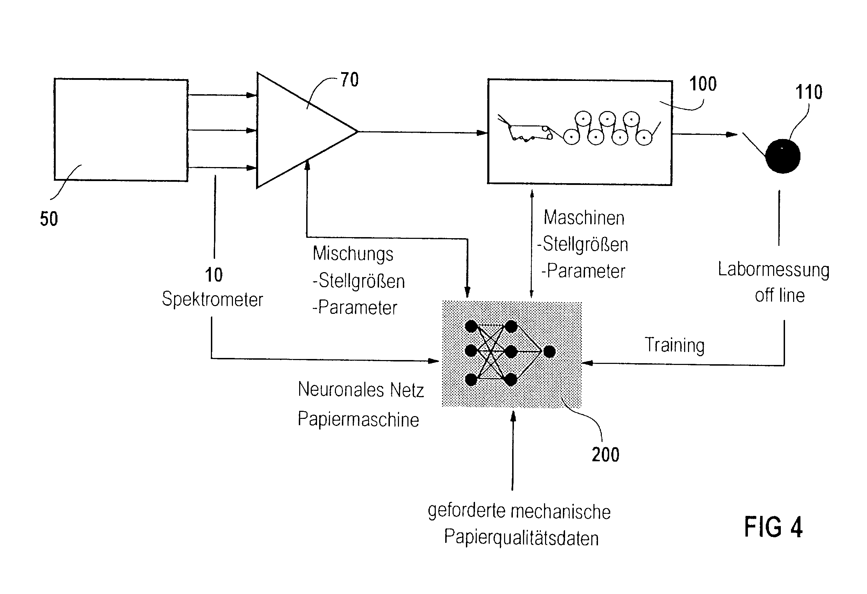

Aus den Darstellungen gemäß den Figuren 4 bis 7 ergeben sich die alternativen Einsatzmöglichkeiten von Spektrometern im Rahmen der Papier- und Kartonherstellung. In den Figuren 4 bis 7 ist jeweils einheitlich eine Einheit 50 zur Stoffaufbereitung, eine sogenannte Stoffzentrale 70, eine Papiermaschine 100, welche der Papiermaschine 1 gemäß FIG entspricht, und ein der Papiermaschine zugeordnetes neuronales Netz 200 vorhanden, welches den neuronalen Netz 20 der FIG 1 entspricht. Die Einheiten 50, 70, 100 und 200 sind in einen Funktionskreis eingebunden.

In FIG 4 ist das Spektrometer 10 gemäß FIG 1 der Einheit 50 zur Stoffaufbereitung zugeordnet. Es können dort beispielsweise Zellstoff oder unterschiedliche Altpapiermateralien erfaßt werden, was durch die die parallelen Pfeile angedeutet ist. Über die Stoffzentrale 70 gelangt geeigneter Ausgangsstoff zur Papiermaschine 100. Dem neuronalen Netz 200 werden neben den Signalen des Spektrometers die Maschinenparameter und die Daten über die geforderte Papierqualität zugeführt. Nach einem Offline-Training über Labormessungen am gefertigten Papier, lassen sich mit den neuronalen Netz 200 die erforderlichen Mischungsparameter für den Ausgangsstoff zur Stoffzentrale 70 geben.

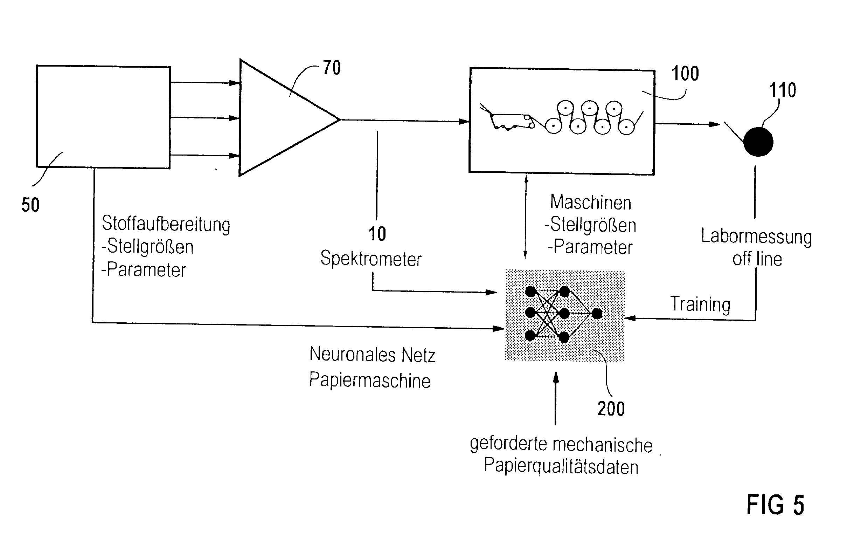

In FIG 5 erfolgt die Messung mit dem Spektrometer 10 beim Stoffeinlauf für die Papiermaschine 100, also nach der Stoffzentrale 70. Bei prinzipiell gleichem Aufbau des neuronalen Netzes 200 ergibt sich hier die Möglichkeit, Stellsignale für die Stoffaufbereitung 50 einerseits und Stellgrößen für die Papiermaschine 100 andererseits zu generieren.

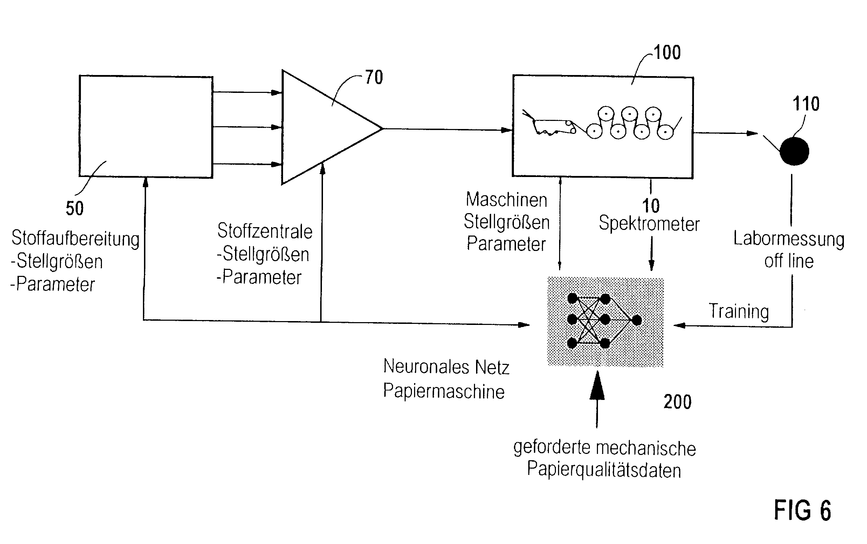

Bei der Anordnung gemäß FIG 6 wird das Spektrometer 10 am bereits gefertigten Papier bzw. Karton, also innerhalb der Papiermaschine 100 eingesetzt. Entsprechend FIG 6 lassen sich mit dem neuronalen Netz ebenfalls Stellgrößen für die Papiermaschine 100 einerseits, aber auch Stellsignale für die Stoffaufbereitung 50 bzw. die Stoffzentrale 70 andererseits gewinnen.

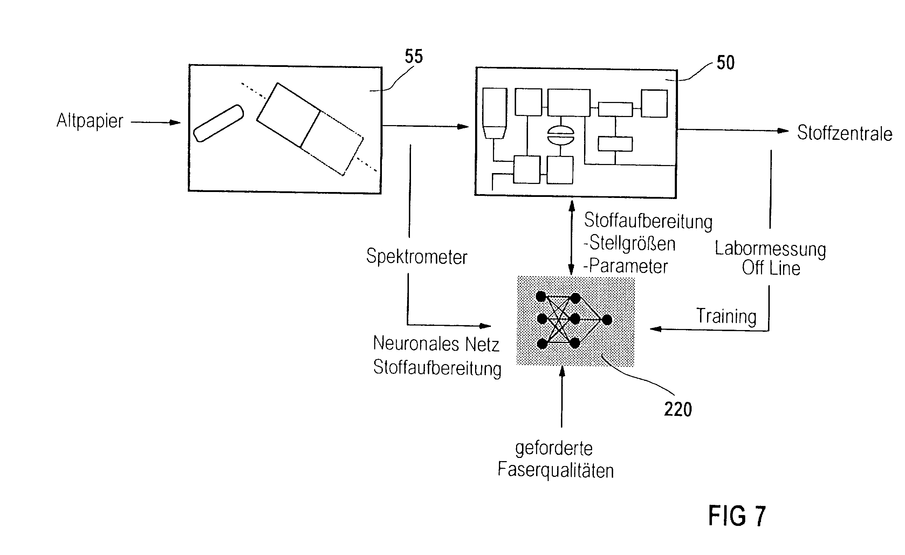

Bei den in den Figuren 4 bis 6 dargestellten Alternativen ist das neuronale Netz 200 jeweils direkt der Papiermaschine 100 zugeordnet, wobei die geforderten mechanischen Papierqualitätsdaten als wesentliche Kenngrößen vorgegeben werden. In FIG 7 ist ein Beispiel angegeben, das speziell die Stoffaufbereitung, also die Einheit 50 der Figuren 4 bis 6, betrifft. Neben der Einheit 50 für die Stoffaufbereitung mit bekannten Einzelelementen, wie einem sogenannten Pulper, einem Refiner od. dgl., ist hier weiterhin eine Einheit für die Stoffauflösung 55 vorhanden, mit der im wesentlichen Altpapier aufbereitet und in bestimmten Anteilen einer geeigneten Zellstoffsuspension zugemischt wird.

In FIG 7 ist der Einheit 50 für die Stoffaufbereitung ein neuronales Netz 220 zugeordnet, dem als Kenngrößen die geforderten Faserqualitäten eingegeben werden. Mit dem Spektrometer 10 wird in diesem Fall vor der eigentlichen Stoffaufbereitung 50 am aufgelösten Stoff gemessen und die gemessenen Intensitätssignale in das neuronale Netz 220 eingegeben. Nach einem geeigneten Training des neuronalen Netzes 220 lassen sich anhand der Meßwerte geeignete Stellsignale für die Stoffaufbereitung gewinnen.

In den einzelnen Beispielen wurde gezeigt, daß durch Online-Messung mit einem oder mehreren Spektrometern und Auswertung mit einem oder mehreren neuronalen Netzen sowie Bestimmung der Qualitätsmaßzahlen des zu fertigenden Papiers bzw. auch Kartons online die qualitätsbestimmenden Parameter der Stoffaufbereitung und der Papiermaschine beeinflußt werden können. Gegenüber den bisherigen diskontinuierlichen Verfahren anhand von Labormessungen entfallen damit störende Verzögerungszeiten.

## Patentansprüche

1. Verfahren zur Prozeßführung einer Papiermaschine für die Papier- und/oder Kartonherstellung unter Verwendung wenigstens einer Meßeinrichtung zur Erfassung physikalischer Kennwerte und einer Regel- oder Steuereinrichtung für die bei der Papiermaschine eingesetzten Betriebsmittel, **dadurch gekennzeichnet,** daß mit der Meßeinrichtung (10,10',10'') spektrale Kennwerte bei unterschiedlichen Wellenlängen an den Betriebsstoffen der Papiermaschine erfaßt werden, wobei die Betriebsstoffe der Ausgangsstoff unmittelbar vor dem Stoffauflauf an der Papiermaschine und/oder die Zwischen- bzw. Endprodukte der Papiermaschine sind, und daß die Signale der Meßeinrichtung (10, 10', 10") für die spektralen Kennwerte von wenigstens einem neuronalen Netz (20, 30, 200, 220) bewertet und daraus Aussagen über die Produktqualität abgeleitet werden, woraus Steuersignale zur Rückwärtsregelung bei der sogenannten Stoffaufbereitung (50) und/oder zur Vorwärtsregelung bei der verwendeten Papiermaschine (100) abgeleitet werden.

2. Verfahren nach Anspruch 1, **dadurch gekennzeichnet**, daß die diffuse Rückstrahlintensität (Iᵢ mit i = 1,...,n) ausgewählter Spektralbereiche gemessen wird.

3. Verfahren nach Anspruch 1, **dadurch gekennzeichnet,** daß die diffuse Durchstrahlintensität ausgewählter Spektralbereiche gemessen wird.

4. Verfahren nach Anspruch 1, unter Verwendung einer Papier- oder Kartonmaschine, **dadurch gekennzeichnet,** daß die der Papiermaschine (1,100) zugeführte Stoffsuspension erfaßt wird.

5. Verfahren nach Anspruch 1, unter Verwendung einer Papier- oder Kartonmaschine, **dadurch gekennzeichnet,** daß das auf der Papiermaschine(1,100) laufende Papier oder Karton erfaßt wird.

6. Verfahren nach Anspruch 4 oder Anspruch 5, **dadurch gekennzeichnet,** daß die Stoffsuspension und/oder das Papier bzw. der Karton kontinuierlich erfaßt werden.

7. Verfahren nach Anspruch 1, **dadurch gekennzeichnet,** daß die neuronalen Netze (20,30,200,220) mit den im Labor gemessenen Qualitätsparametern des Papiers oder des Kartons trainiert werden.

8. Verfahren nach einem der vorhergehenden Ansprüche, mit einer Stoffzentrale zur Stoffaufbereitung für die Papier- und Kartonherstellung, **dadurch gekennzeichnet,** daß die Stoffmischung in der Stoffzentrale (70) entsprechend den spektralen Kenngrößen der einzelnen Ströme oder anhand des Gesamtspektrums der die Stoffzentrale (70) verlassenden Stoffe erfolgt.

9. Vorrichtung zur Durchführung des Verfahrens nach Anspruch 1 oder einem der Ansprüche 2 bis 10, mit wenigstens einer Meßeinrichtung zur Erfassung spektraler und/oder physikalischer Kennwerte und wenigstens einer Regel- oder Steuereinrichtung für die eingesetzten Betriebsmittel, wobei die Meßeinrichtung für die spektralen Kennwerte ein Spektrometer (10,10',10'') zur Erfassung von intensitätsmeßwerten (Iᵢ) bei unterschiedlichen Wellenlängen (λᵢ) ist, von denen durch geeignete Auswertung Korrektursignale für die Regel- oder Steuereinrichtung ableitbar sind und daß zur Auswertung wenigstens ein neuronales Netz (20,30,220,230) vorhanden ist.

## Claims

1. Method for the process management of a paper machine for the production of paper and/or board, using at least one measuring device for registering physical characteristic values and a regulating or controlling device for the operating means used in the paper machine, characterized in that, using the measuring device (10, 10', 10"), spectral characteristic values are registered at different wavelengths on the operating materials of the paper machine, the operating materials being the starting material directly before the flow box on the paper machine and/or the intermediate or final products of the paper machine, and in that the signals from the measuring device (10, 10', 10") for the spectral characteristic values are evaluated by at least one neural network (20, 30, 200, 220) and that statements about the product quality are derived therefrom, and from these, control signals for feedback control in the so-called stock preparation (50) and/or for feedforward control in the paper machine (100) used are derived.

2. Method according to Claim 1, characterized in that the diffuse backscatter intensity (Iᵢ with i = 1, ...., n) of selected spectral regions is measured.

3. Method according to Claim 1, characterized in that the diffuse transmitted intensity of selected spectral regions is measured.

4. Method according to Claim 1, using a paper or board machine, characterized in that the stock suspension fed to the paper machine (1, 100) is registered.

5. Method according to Claim 1, using a paper or board machine, characterized in that the paper or board running on the paper machine (1, 100) is registered.

6. Method according to Claim 4 or Claim 5, characterized in that the stock suspension and/or the paper or the board are registered continuously.

7. Method according to Claim 1, characterized in that the neural networks (20, 30, 200, 220) are trained using the quality parameters of the paper or of the board measured in the laboratory.

8. Method according to one of the preceding claims, having a central stock area for stock preparation for the paper and board production, characterized in that the stock mixing in the central stock area (70) is carried out in accordance with the spectral characteristic variables of the individual streams or with reference to the overall spectrum of the stocks leaving the central stock area (70).

9. Device for carrying out the method according to Claim 1 or one of Claims 2 to 8, having at least one measuring device for registering spectral and/or physical characteristic values and at least one regulating or controlling device for the operating means used, the measuring device for the spectral characteristic values being a spectrometer (10, 10', 10") for registering intensity measured values (Iᵢ) at different wavelengths (λᵢ), from which correction signals for the regulating or controlling device can be derived by means of suitable evaluation, and in that there is at least one neural network (20,30,220,230) for the evaluation.

## Revendications

1. Procédé de commande du processus d'une machine à papier pour la fabrication de papier et/ou de carton, en utilisant au moins un dispositif de mesure pour relever des valeurs caractéristiques physiques et un dispositif de régulation et/ou de commande des moyens d'exploitation utilisés dans la machine à papier, caractérisé en ce que l'on relève par le dispositif (10, 10', 10") de mesure des valeurs caractéristiques spectrales à différentes longueurs d'onde sur les matières d'exploitation de la machine à papier, les matières d'exploitation étant la matière de départ juste avant l'arrivée de la pâte à la machine à papier et/ou les produits intermédiaires ou finals de la machine à papier, et en ce que l'on évalue les signaux du dispositif (10, 10', 10") de mesure pour les valeurs caractéristiques spectrales par au moins un réseau (20, 30, 200, 220) neuronal et en ce que l'on en déduit des prévisions sur la qualité du produit, des signaux de commande pour la régulation en amont dans ce que l'on appelle la préparation (50) de la pâte et/ou pour la régulation en aval dans la machine (100) à papier utilisée en étant déduits.

2. Procédé suivant la revendication 1, caractérisé en ce que l'on mesure l'intensité (Iᵢ avec i = 1,..., n) du rayonnement rétrodiffusé dans des domaines spectraux sélectionnés.

3. Procédé suivant la revendication 1, caractérisé en ce que l'on mesure l'intensité du rayonnement diffusé par transmission dans des domaines spectraux sélectionnés.

4. Procédé suivant la revendication 1, avec utilisation d'une machine à papier ou à carton, caractérisé en ce que l'on effectue un relevé sur la suspension de pâte envoyée à la machine (1, 100) à papier.

5. Procédé suivant la revendication 1, avec utilisation d'une machine à papier ou à carton, caractérisé en ce que l'on effectue un relevé sur le papier ou le carton passant dans la machine (1, 100) à papier.

6. Procédé suivant la revendication 4 ou la revendication 5, caractérisé en ce que l'on effectue un relevé en continu sur la suspension de pâte et/ou sur le papier ou le carton.

7. Procédé suivant la revendication 1, caractérisé en ce que l'on donne aux réseaux (20, 30, 200, 220) neuronaux un apprentissage par les paramètres de qualité du papier ou du carton mesurés en laboratoire.

8. Procédé suivant l'une des revendications précédentes, comportant une centrale de pâte pour la préparation de la pâte pour la fabrication de papier et/ou de carton, caractérisé en ce que le mélange de matière dans la centrale (70) de pâte s'effectue suivant les grandeurs caractéristiques spectrales des courants individuels ou à l'aide de tout le spectre des matières quittant la centrale (70) de pâte.

9. Dispositif pour mettre en oeuvre le procédé suivant la revendication 1 ou l'une des revendications 2 à 10, comportant au moins un dispositif de mesure pour relever des valeurs caractéristiques spectrales et/ou physiques et au moins un dispositif de régulation et/ou de commande des moyens d'exploitation utilisés, le dispositif de mesure des valeurs caractéristiques spectrales étant un spectromètre (10, 10', 10") pour relever des valeurs (Iᵢ) de mesure d'intensité à différentes longueurs (λᵢ) d'onde, dont il peut être déduit, par une exploitation appropriée, des signaux de correction pour le dispositif de régulation et/ou de commande, et au moins un réseau (20, 30, 220, 230) neuronal étant présent pour l'exploitation.
